# EUROPÄISCHE PATENTSCHRIFT

(11) **EP 2 058 013 B1**
(45) Veröffentlichungstag und Bekanntmachung des Hinweises auf die Patenterteilung: **27.01.2016**
(21) Anmeldenummer: 08019752.8
(22) Anmeldetag: 12.11.2008
(51) Int. Cl.: A61L 9/12, B60H 3/00

(54) **Beduftungsvorrichtung**
Scenting device
Dispositif d'embaumement

(30) Priorität: 12.11.2007 DE 102007054166
(43) Veröffentlichungstag der Anmeldung: 13.05.2009
(73) Patentinhaber: MAHLE Behr GmbH & Co. KG, 70469 Stuttgart (DE)
(72) Erfinder: Fieger, Martin, 71732 Tamm (DE); Fritsche, Uwe, 71686 Remseck am Neckar (DE); Zeller, Kuno, 70469 Stuttgart (DE); D'Angelo, Marco, 70469 Stuttgart (DE); Tidelski, Axel, 70771 Leinfelden-Echterdingen (DE); Paul, Michael, 74343 Sachsenheim (DE)
(74) Vertreter: Grauel, Andreas

(56) Entgegenhaltungen:
- CA-A1- 2 495 470
- GB-A- 2 247 623
- GB-A- 2 417 422
- US-A1- 2002 197 189
- US-A1- 2006 196 100

## Beschreibung

### Beduftungsvorrichtung

Die Erfindung betrifft eine Beduftungsvorrichtung gemäß dem Anspruch 1.

Aus der DE 103 27 122 A1 ist eine Belüftungsvorrichtung, insbesondere zum Beduften eines Kraftfahrzeugs, bekannt, welche einen Duftstoff enthält, mit einer Dosiereinrichtung, wobei die Beduftungsvorrichtung zumindest einen Hohlkörper, der mit mindestens einer Öffnung versehen ist, und ein Element, insbesondere einen Hohlkörper, mit mindestens einer weiteren Öffnung umfasst. Dabei sind der Hohlkörper und/oder das Element mittels eines Motors derart antreibbar, dass eine Relativbewegung zwischen dem Hohlkörper und dem Element möglich ist, so dass die beiden Öffnungen in zumindest einer Stellung der Relativbewegung ein Austreten von Duftstoff von innen nach außen ermöglichen. Der Duftstoff selbst ist innerhalb des Hohlkörpers in einer Duftstoffpatrone oder -kartusche angeordnet, die bei Bedarf auswechselbar ist.

Eine andere Beduftungsvorrichtung ist aus der US 5,314,669 A bekannt. Bei dieser Beduftungsvorrichtung sind zwei in axialer Richtung zueinander angeordnete Hohlzylinder in einem im Wesentlichen als Hohlzylinder ausgebildeten Gehäuse drehbar aufgenommen. Der das Gehäuse bildende Hohlzylinder weist zwei rechteckförmige, sich in Längsrichtung erstreckenden Öffnungen in seiner Mantelfläche auf einander gegenüberliegenden Seiten auf, die sich jeweils zumindest über einen Teilbereich der inneren Hohlkörper erstrecken. Die inneren Hohlzylinder weisen ebenfalls jeweils zwei einander gegenüberliegende Öffnungen in ihren Mantelflächen auf, die jedoch gestuft ausgebildet sind, d.h. die Form dreier jeweils um die Hälfte ihrer Länge versetzt zueinander angeordneter, langgestreckter und zusammen eine gemeinsame Öffnung bildender Rechtecke aufweisen. Die Öffnungen der inneren Hohlzylinder sind verdreht zueinander angeordnet, so dass verschiedene Beduftungsvariationen möglich sind.

Die DE 10 2005 041 986 A1 offenbart ein Duftstoffgerät zur Anwendung in einem Kraftfahrzeug mit einem Gehäuse zur Aufnahme eines Duftstoffs, sowie einem Ventilator mit einem Antrieb, welcher einen Exzenteraufzug und eine Speicherfeder zum Speichern der Energie des Exzenteraufzugs aufweist. Ähnlich der Energiespeicherung bei mechanischen, automatischen Armbanduhren wird die Bewegung eines Fahrzeugs, insbesondere das Beschleunigen oder Verzögern zur Betätigung des Exzenteraufzugs und damit zum Aufziehen der Speicherfeder genutzt. Der Ventilator des Duftstoffgeräts kann somit laufen, wenn das Fahrzeug entsprechende Bewegungsenergie zur Verfügung stellt bzw. solange bei einem beispielsweise abgestelltem Fahrzeug Energie in der Feder gespeichert ist.

Derartige Beduftungsgeräte sind somit vom Fahrbetrieb abhängig und lassen insbesondere bei einem längeren Fahrzeugstillstand noch Wünsche offen.

Eine Beduftungsvorrichtung für ein Kraftfahrzeug gemäß dem Oberbegriff des Anspruchs 1 ist aus US 2006/0196100 A1 bekannt.

Es ist Aufgabe der Erfindung, eine verbesserte Beduftungsvorrichtung zur Verfügung zu stellen.

Diese Aufgabe wird gelöst durch eine Beduftungsvorrichtung mit den Merkmalen des Anspruchs 1. Vorteilhafte Ausgestaltungen sind Gegenstand der Unteransprüche.

Hierbei ist eine Beduftungsvorrichtung für ein Kraftfahrzeug vorgesehen, mit zumindest einer Duftstoffaufnahmevorrichtung, einem Lüfterrad, einem Antriebsmotor für das Lüfterrad und einer elektrischen Energieversorgungseinrichtung für den Antriebsmotor des Lüfterrads, die in einem Gehäuse angeordnet beziehungsweise integriert ausgebildet sind. Dabei versorgt erfindungsgemäß die Energieversorgungseinrichtung für den Antriebsmotor des Lüfters, den Antriebsmotor unabhängig vom Energieversorgungssystem des Kraftfahrzeugs mit elektrischer Energie. Ein derartiges System hat einen sehr einfachen und kompakten Aufbau und ist unabhängig vom Fahrzustand des Fahrzeugs oder vom Ladezustand der Fahrzeugbatterie betreibbar. Insbesondere bei längeren Stillstandszeiten eines Fahrzeugs wie beispielsweise in einem Stau lässt sich für den Fahrgast ein angenehmes Raumklima durch entsprechende Beduftung erzielen ohne die Fahrzeugbatterie zu belasten. Insbesondere lässt sich die erfindungsgemäße Beduftungsvorrichtung durch ihre autarke Energieversorgung und Kompaktheit vorteilhaft als Nachrüstlösung unabhängig vom Fahrzeugtyp einsetzen.

Vorteilhafterweise umfasst die Energieversorgungseinrichtung zumindest eine oder mehrere Batterien, die bevorzugt wiederaufladbar sind. Insbesondere kann eine aus mehreren Batterien oder Akkumulatoren bestehende Baugruppe vormontiert in die Beduftungsvorrichtung eingesetzt werden.

Bevorzugt ist im Gehäuse der Beduftungsvorrichtung eine Steuereinheit für den Antriebsmotor des Lüfterrades angeordnet. Die Steuereinheit wird dabei ebenfalls von der Energieversorgungseinrichtung, wie beispielsweise den Batterien mit Spannung gespeist und umfasst besonders vorteilhaft eine Drehzahlregelung für den Lüftermotor. Insbesondere ist die Steuereinheit mit einem Zeitschalter oder einem so genannten Timer ausgestattet mittels welchem ein Zeitintervall für den Betrieb des Antriebsmotors festlegbar ist. Alternativ oder zusätzlich ist ein Bewegungsmelder an der Beduftungsvorrichtung oder der Steuereinheit integriert, dessen Signal ebenfalls in der Steuereinheit verarbeitbar ist. Vorteilhafterweise, kann bei einem Fehlen von detektierten Bewegungen in einem Fahrzeug, wie dies insbesondere bei abgestelltem Fahrzeug ohne Fahrzeuginsassen der Fall ist, ein Abschaltsignal für den Antriebsmotor generiert werden.

Am Gehäuse der Beduftungsvorrichtung ist weiter bevorzugt ein Anschluss für ein Ladekabel vorgesehen, mittels welchem die wiederaufladbaren Batterien geladen werden können. Insbesondere ist dieses Ladekabel mit einem Stecker für den Zigarettenanzünder versehen. Ein an den Zigarettenanzünder angeschlossenes Ladekabel ist, ausgerüstet mit einer Messvorrichtung, bevorzugt auch zur Messung der aktuellen Ladespannung geeignet, wobei eine Ladespannung Null, wie Sie in vielen Fällen bei ausgeschalteter Zündung am Zigarettenanzünder vorliegt, ein Abschaltsignal für die Steuereinheit und damit für den Antriebsmotor des Lüfters der Beduftungsvorrichtung erzeugt.

Bevorzugt ist das Gehäuse der Beduftungsvorrichtung im Wesentlichen zylindrisch ausgeführt. Die Beduftungsvorrichtung lässt sich so beispielsweise auf einfache Weise in einem so genannten Becherhalter oder Cupholder eines Fahrzeugs einsetzen, ohne dass beispielsweise bei der Nachrüstung eines bestehenden Fahrzeugs mit einer Beduftungsvorrichtung eine zusätzliche Aufnahme im Fahrzeug montiert bzw. zur Verfügung gestellt werden muss.

Zur einfachen Wartung beziehungsweise zum Austausch von Duftstoffpatronen oder Duftstoffkartuschen weist das Gehäuse vorteilhafterweise eine Abdeckung auf, die insbesondere durch eine Schraub- und/oder Schnappverbindung am Gehäuse bzw. an einem Gehäuseunterteil montier- und demontierbar ist.

Die Duftstoffaufnahmevorrichtung umfasst eine im Gehäuse der Beduftungsvorrichtung feststehende Einfassung und einen, insbesondere im Gehäuse drehbar angeordneten Aufnehmer, welcher insbesondere ein Aufnahmefach oder mehrere Aufnahmefächer für einen oder mehrere Duftstoffkartuschen aufweist.

Um den ungewollten Austrag von Duftstoff zu vermeiden, sind in einem Bereich zwischen dem Aufnehmer und der Einfassung ein oder mehrere Dichtelemente angeordnet.

Das Gehäuse der Beduftungsvorrichtung weist bevorzugt ein Unterteil auf, das mit der Abdeckung über einen Verbindungsring, der vorteilhaft als Gewindering ausgebildet ist, verbunden ist.

Weiter bevorzugt ist die Intensität des Duftaustrags durch Verdrehung der Abdeckung einstellbar.

Im Folgenden wird die Erfindung anhand von Ausführungsbeispielen, teilweise unter Bezugnahme auf die Zeichnung, im Einzelnen erläutert. Es zeigen:
- Fig. 1: eine perspektivische Darstellung der Beduftungsvorrichtung gemäß dem erfindungsgemäßen ersten Ausführungsbeispiel,
- Fig. 2: eine Seitenansicht der Beduftungsvorrichtung gemäß Fig.1,
- Fig. 3: einen Schnitt entlang der Linie A-A gemäß Fig.2,
- Fig. 4: eine perspektivische Darstellung der Beduftungsvorrichtung ohne äußeres Gehäuse,
- Fig. 5: eine perspektivische Darstellung der Beduftungsvorrichtung ohne äußeres Gehäuse und ohne Abdeckung,
- Fig. 6: eine Darstellung der Anordnung der erfindungsgemäßen Beduftungsvorrichtung in einem Becherhalter eines Fahrzeugs,
- Fig. 7: eine Ansicht einer Beduftungsvorrichtung gemäß einem erfindungsgemäßen zweiten Ausführungsbeispiel,
- Fig. 8: einen Schnitt entlang der Linie C-C gemäß Fig.7,
- Fig. 9: einen Schnitt entlang der Linie B-B gemäß Fig.7,
- Fig. 10: einen Schnitt entlang der Linie D-D gemäß Fig.7,
- Fig. 11: eine perspektivische Darstellung der Abdeckung der Beduftungsvorrichtung gemäß dem zweiten Ausführungsbeispiel und
- Fig. 12: eine perspektivische Darstellung der Beduftungsvorrichtung ohne Abdeckung gemäß dem zweiten Ausführungsbeispiel.

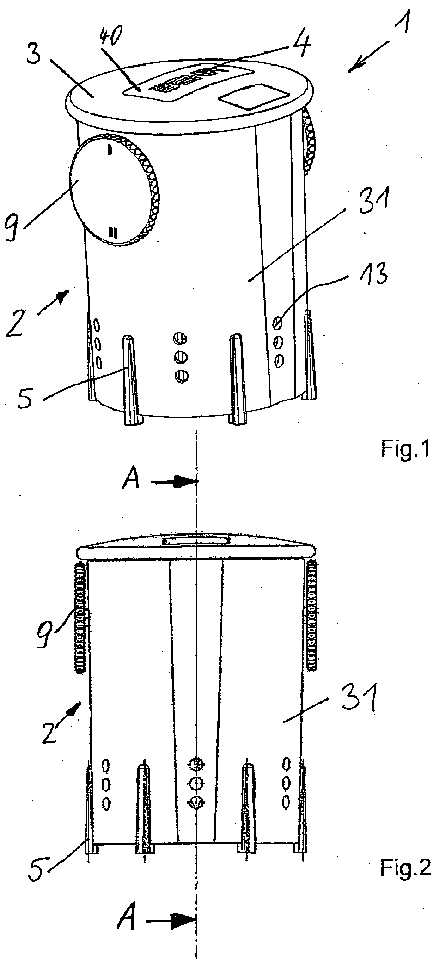

Eine Beduftungsvorrichtung 1 gemäß dem in Figur 1 dargestelltem Ausführungsbeispiel weist ein Gehäuse 2 auf, das im Wesentlichen zylindrisch ausgebildet ist. An einem oberen Teil des Gehäuseunterteils 31 ist eine Abdeckung 3 mit einer Öffnung 4 vorgesehen, wobei die Abdeckung mittels einer Schnappverbindung an dem Gehäuseunterteil 31 befestigt ist. Alternativ ist es auch denkbar, dass die Abdeckung mittels einer Schwenkverbindung, beispielsweise einem Filmscharnier oder einer kombinierten Rast- Schwenkverbindung am Gehäuseunterteil befestigbar ist. Die Abdeckung 3 kann so in einfacher Weise am Gehäuseunterteil 31, insbesondere ohne Zuhilfenahme eines Werkzeugs montiert und demontiert werden.

Die Öffnung 4 ist mit einer aus transparentem Kunststoff bestehenden Platte 40 versehen in welcher sich die eigentliche Durchtrittsöffnung 4 für austretende mit Duftstoff versehene Luft befindet, und vorliegend beispielsweise in Form eines Schriftzuges oder Firmenlogos ausgebildet ist. Mittels der aus transparentem Kunststoff bestehenden Öffnung ist, wie in den weiteren Figuren gezeigt, ein direkter Blick auf eine im Inneren der Beduftungsvorrichtung 1 angeordnete Duftkartusche 14 möglich. Neben der Platte 40 ist es auch denkbar die gesamte Abdeckung 3 aus transparentem Kunststoff auszubilden. An dem der Abdeckung 3 gegenüberliegenden Ende der Beduftungsvorrichtung 1 sind entlang des Umfangs des Gehäuses 2 stabförmige Dämpfungselemente 5 vorgesehen, die zur Aufnahme in einem Becherhalter eines Fahrzeugs und zur Entkopplung und Schwingungsdämpfung zwischen dem Gehäuse 2 und dem Becherhalter 22 und damit dem Cockpit des Fahrzeugs dienen. Die Dämpfungselemente bestehen aus einem elastomeren Kunststoff und sind bevorzugt mittel eines Zwei-Komponenten-Spritzgusses gemeinsam mit dem Gehäuseunterteil 31 hergestellt. Über die Ansaugöffnungen 13 und eine Öffnung im Bodenbereich des Gehäuseunterteils 31 kann Luft in das Gehäuse 2 zur Weiterbeförderung eintreten.

Fig. 2 zeigt eine Seitenansicht der in Fig.1 dargestellten Beduftungsvorrichtung 1, wobei zu erkennen ist, dass sechs Dämpfungselemente 5, von denen vier in der Ansicht zu sehen sind, entlang des Umfangs des unteren Bereichs des Gehäuses 2 angeordnet sind. Der durch diese Dämpfungselemente 5 beschriebene Umkreis weist einen etwas größeren Durchmesser auf als die entsprechende Aufnahme eines Becherhalters 22, so dass es beim Einführen der Beduftungsvorrichtung 1 in den Becherhalter 22 zu einem leichten Deformieren dieser Dämpfungselemente 5 kommt, wodurch die Beduftungsvorrichtung 1 sicher im Becherhalter 22 fixiert wird.

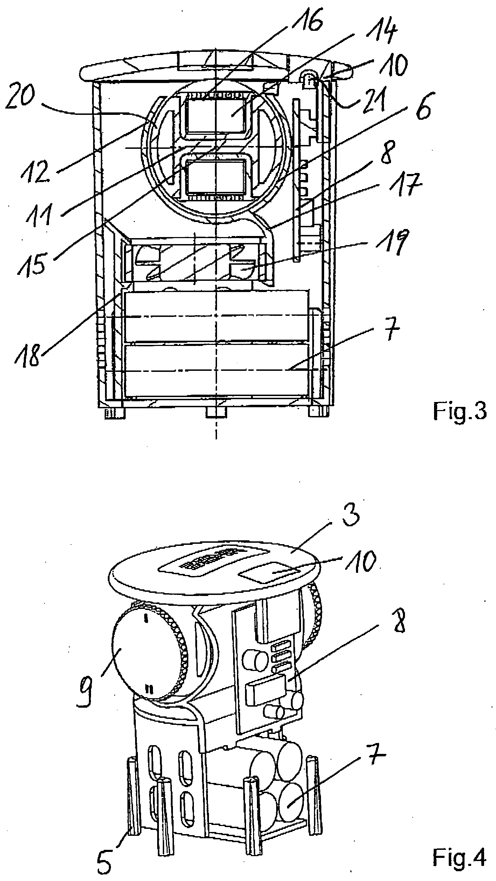

Der innere Aufbau und die Funktionsweise der Beduftungsvorrichtung 1 wird anhand des in Fig. 3 dargestellten Schnittbildes näher erläutert. Die Duftstoffaufnahmevorrichtung 6 umfasst dabei die Einfassung 12 in welcher der drehbar angeordnete Aufnehmer 11, vorliegend für zwei Duftkartuschen 14 geführt ist. Die Kartuschen 14 sind im gezeigten Ausführungsbeispiel quaderförmig ausgeführt und auf der, der Drehachse 15 des Aufnehmers 11 abgewandten Seite mit einem luftdurchlässigen Gitter 16 versehen. Der Duftstoff befindet sich im Inneren der Kartusche und wird in Form eines duftstoffgetränkten Vlieses oder einer körnerartigen Substanz, deren Korngröße größer als der Gitterabstand des Gitters 16 ist, insbesondere größer als 2 mm ist, zur Verfügung gestellt. Ein Tausch einer verbrauchten Duftsstoffkartusche 14 kann auf einfache Weise nach Entfernen der wiedermontierbaren Abdeckung 3 erfolgen.

Die Einfassung 12 der Duftstoffaufnahmeeinrichtung 6 ist als oben offener Hohlzylinder ausgebildet. Der Öffnungswinkel im Schnittbild beträgt vorliegend etwa 90°. Anschließend an diesen Hohlzylinder ist ein Aufnahmeschenkel 17 vorgesehen welcher im Zusammenspiel mit einem inneren Gehäuse 18 der Aufnahme des Lüfterrades 19 und dem nicht explizit dargestellten Antriebsmotor für das Lüfterrad dient. Gleichzeitig fungiert dieser Aufnahmeschenkel 17 auch als Luftleitelement für den vom Lüfterrad 19 geförderten Luftstrom.

In der in Fig. 3 gezeigten Stellung des Aufnehmers 11 strömt der vom Lüfterrad 19 geförderte Luftstrom auf der linken Seite an der Einfassung 12 vorbei nach oben und fördert bei eingeschaltetem Antriebsmotor des Lüfterrades 19 die oberhalb oder im Bereich des Gitters 16 der Duftstoffkartusche 14 befindlichen Duftstoffteilchen oder -moleküle über die Öffnung in der Austrittsöffnung 4 nach außen und damit in den Innenraum des entsprechenden Kraftfahrzeugs.

Die Intensität dieses mit Duftstoffen beladenen Luftstroms wird mittels der Verdrehung des Bedienrades 9 in fester Kopplung mit dem Aufnehmer 11 bestimmt. Das heißt, dass durch Verdrehen des Aufnehmers 11, die mit dem Bereich der Öffnung der Einfassung 12 zur Deckung kommende Querschnittsfläche des Gitters 16 der Duftkartusche 14 bestimmt wird. Dreht man den Aufnehmer 11 beispielsweise um 90° aus der in Fig. 3 dargestellten Stellung, so verschließt der Bereich 20 des Aufnehmers 11 die Öffnung der Einfassung vollständig und es kann auch bei eingeschaltetem Lüfter kein Duftstoff aus der Kartusche ausgetragen werden. Um dies zu gewährleisten sind zudem entweder an der Einfassung 12 oder den Bereichen 20 des Aufnehmers 11 nicht dargestellte Dichtelemente vorgesehen, die den Zwischenraum zwischen den Bereichen 20 und der Einfassung 12 luftdicht verschließen.

Dreht man den Aufnehmer 11 um weitere 90°, so kommt die zweite Kartusche 14 zum Einsatz, bzw. vor der Öffnung der Einfassung 12 zu stehen. In der zweiten Duftkartusche 14 befindet sich vorliegend ein zweiter, vom Duftstoff in der ersten Duftkartusche 14 verschiedener Duftstoff. Alternativ kann die zweite Duftkartusche 14 auch als Ersatzkartusche dienen. Die beiden um 180° zueinander verdrehten Stellungen der Duftkartuschen 14 sind an dem Bedienrad 9 als Stellung "I" und Stellung "II" markiert, wie aus Fig. 1 ersichtlich. Selbstverständlich ist die Erfindung nicht auf Aufnehmer 11 mit zwei sich gegenüberliegenden Duftkartuschen 14 beschränkt, sondern es ist auch eine Anzahl von drei, vier oder mehr Duftkartuschen denkbar.

Mittels der dargestellten Ausführungsform kann sowohl der Aufnehmer 11 mit den beiden Duftkartuschen 14, als auch das Lüfterrad 19 mit dem Antriebsmotor und den Batterien der Energieversorgungseinrichtung 7 mit Hilfe der Einfassung 12 und dem inneren Gehäuse 18 zu einer Baugruppe vormontiert werden, die in das Gehäuseunterteil 31 eingesetzt und an diesem befestigt wird.

Die Drehung des Aufnehmers 11 ist mittels des Bedienrades 9 von außerhalb des Gehäuses 2 durchführbar, so dass mittels dieses Bedienrades 9 die Intensität des Duftaustrags beziehungsweise die Wahl eines Duftstoffes gesteuert werden kann. Zudem ist seitlich der Einfassung 12 die Steuereinheit 8 für den Antriebsmotor des Lüfters angeordnet. Mittels eines Schalters 10 an der Oberseite der Abdeckung 3 wird die Steuereinheit 8 bedient, wobei dieser Schalter in einer einfachen Variante als Ein/Aus-Schalter ausgeführt ist oder als mehrstufiger Schalter die Wahl verschiedener Drehzahlen des Lüfters gestattet. Vorliegend ist der Schalter 10 ebenfalls aus transparentem Kunststoff ausgeführt, so dass das Leuchten von LEDs 21, die den Betriebszustand der Beduftungsvorrichtung anzeigen, von außen wahrgenommen werden kann. Ebenfalls in die Steuereinheit integriert ist ein Timer bzw. Zeitschalter, an welchem ein Wert für eine Zeitspanne, nach welcher eine automatische Abschaltung des Antriebsmotors des Lüfterrades 19 erfolgt, eingestellt werden kann. Die Einstellung dieses Wertes für das entsprechende Zeitintervall kann bei Auslieferung der Beduftungsvorrichtung voreingestellt oder vom entsprechenden Benutzer vorgenommen werden. Vorzugsweise wird ein Wert von ein bis fünf Stunden gewählt. Dadurch wird insbesondere verhindert, dass eine unbeabsichtigt in Betrieb gebliebene Beduftungsvorrichtung die Batterien unnötig belastet und/oder dem Fahrzeuginnenraum eine als unangenehm empfundene Duftsstoffkonzentration zugeführt und die Duftstoffkartuschen 14 vorzeitig entleert werden.

Ebenfalls zur Verhinderung eines nicht erforderlichen Betriebs der Beduftungsvorrichtung 1, dient das Signal eines Bewegungsmelders (in den Fig. nicht dargestellt), der in die Steuereinheit 8 oder das Gehäuse 2 der Beduftungsvorrichtung integriert ist. Misst dieser Bewegungsmelder für einen definierten Zeitraum kein Signal einer Bewegung, so kann darauf geschlossen werden, dass sich im Fahrzeug keine Personen befinden und das Fahrzeug abgestellt ist, so dass die Steuereinheit ein Signal zur Abschaltung des Antriebsmotors des Lüfterrades 19 generiert und diesen abschaltet.

Fig. 4 verdeutlicht in einer Darstellung der Beduftungsvorrichtung 1 ohne das in Fig.1 gezeigte Gehäuse 2 nochmals den inneren Aufbau der Vorrichtung. Andeutungsweise ist die Position der Dämpfungselemente 5 am unteren Bereich zu erkennen.

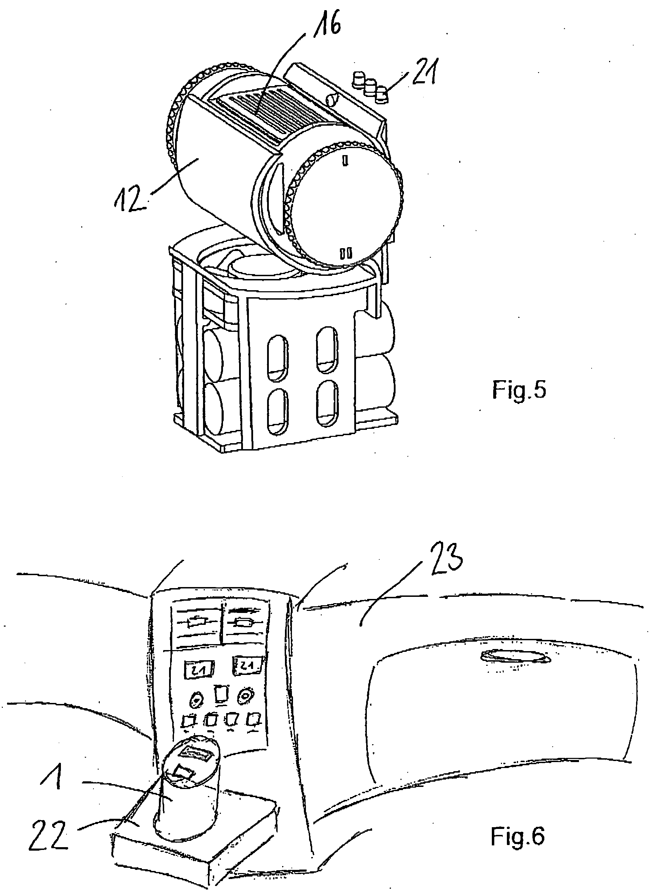

Fig. 5 zeigt die Beduftungsvorrichtung in ähnlicher Weise wie in Fig. 4, wobei in der Darstellung die Abdeckung 3 entfernt wurde. Schematisch sind die drei Leuchtdioden 21 zur Signalisierung des Betriebszustandes der Beduftungsvorrichtung 1 bzw. des Antriebsmotors des Lüfterrades 19 zu erkennen. Im offenen Bereich der hohlzylindrischen Einfassung 12 ist das Gitter 16 der Duftkartusche 14 zu sehen.

Fig. 6 zeigt die Beduftungsvorrichtung 1 in einem Becherhalter 22 im Frontbereich einer Instrumententafel 23 eines Kraftfahrzeugs. Aufgrund der autarken Energieversorgung ist die Anordnung der Beduftungsvorrichtung 1 auch an beliebigen Positionen, wie beispielsweise auf der Hutablage im Fahrzeuginnenraum möglich.

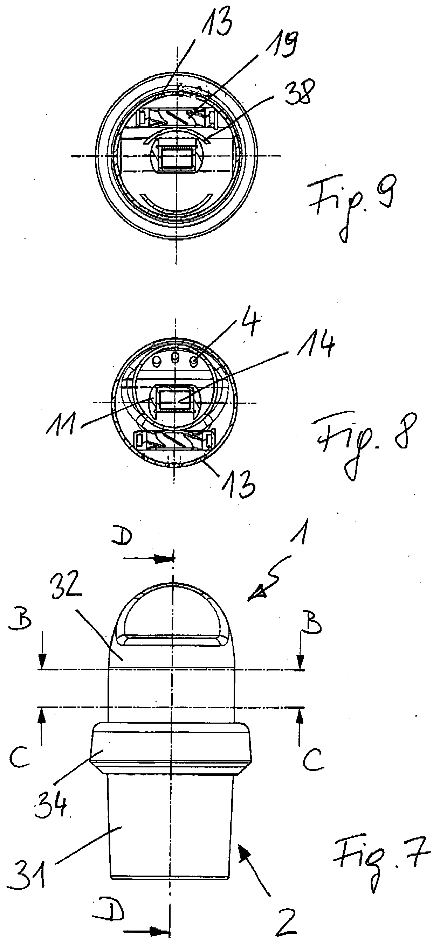

In den Figuren 7 bis 12 ist ein zweites Ausführungsbeispiel dargestellt, wobei für funktionell gleiche Teile wie im ersten Ausführungsbeispiel dieselben Bezugszeichen verwendet wurden.

Eine Ansicht der Beduftungsvorrichtung 1 gemäß dem zweiten Ausführungsbeispiel ist in Fig.7 dargestellt. Das Gehäuse 2 der Beduftungsvorrichtung 1 wird dabei von einem Unterteil 31 und einer Abdeckung 32 gebildet, in welchem alle Komponenten der Beduftungsvorrichtung aufgenommen sind. Verbunden sind das Unterteil 31 und die Abdeckung 32 über einen Gewindering 34.

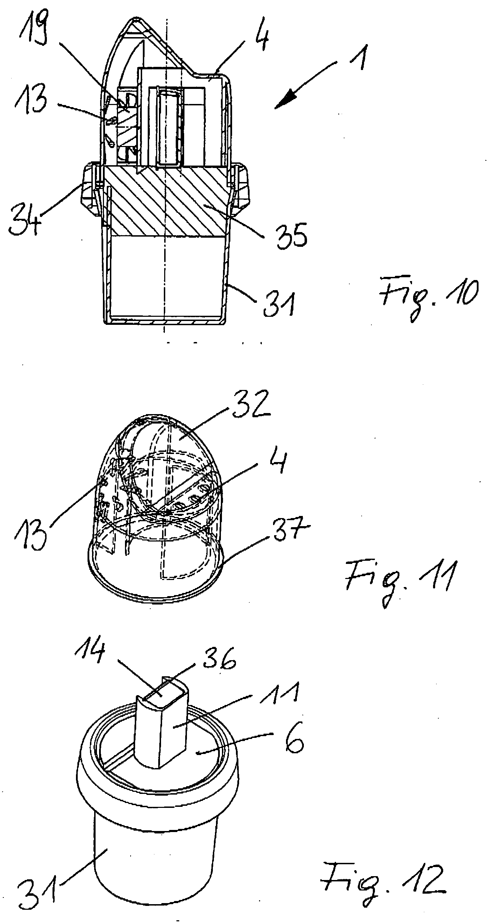

In der Schnittdarstellung nach Fig. 10, die einem Schnitt D-D gemäß Fig.7 entspricht, ist zu erkennen, dass das Unterteil 31 in der Form eines Bechers mit einem Träger 35 ausgebildet ist, Der Träger 35 bildet die Duftaufnahmevorrichtung 6, wobei ein Aufnehmer 11 in Form eines tonnenförmigen Hohlkörpers, der nach einer Seite hin offen ist, vorgesehen ist. Im im wesentlichen quaderförmig ausgebildeten Hohlraum im Aufnehmer 11 sind an zwei Innenseiten Schlitze 36 ausgebildet, welche der Aufnahme einer Duftkartusche 14 dienen. Die Duftkartusche 14 wird in den Aufnehmer eingeschoben, wobei entsprechende leistenförmige Vorsprünge an der Duftkartusche 14 in den Schlitzen 36 geführt werden. Der Aufnehmer 11 und der Träger 35 sind vorliegend einstückig ausgebildet.

Wie in Fig. 12 dargestellt, bilden das Unterteil 31, der Träger 35 und der Aufnehmer 11 eine Baueinheit. Zusätzlich ist in Fig. 12 auch der mit dem Unterteil 31 verschraubte Gewindering 34 zu erkennen, wobei das Zusammenwirken mit der Abdeckung 32 aus Fig. 10 hervorgeht. Hierbei wird die Abdeckung 32 auf das Unterteil 31, bzw. den Träger 35 aufgesetzt. Anschließend wird der Gewindering 34 über die Abdeckung geschoben und mit dem Unterteil 31 an welchem ein entsprechendes Außengewinde vorgesehen ist, verschraubt. Die Abdeckung 32 weist an ihrem unteren Ende einen Flansch 37 auf, welcher von dem Gewindering 34 umfasst wird, wobei ein geringfügiger Abstand, der gegebenenfalls mit einem elastischen Dichtmittel abgedichtet wird, verbleibt, so dass die Abdeckung 32 gegenüber dem Unterteil 31 verdrehbar ist.

Der Lüfter 19 und der Motor (in den Figuren nicht dargestellt) ist seitlich an der Abdeckung 32 gehaltert, saugt Luft über sternförmige Öffnungsschlitze 13 an und fördert diese um eine Prallwand 38 herum, wobei sie mit dem Duftstoff aus der Kartusche 14 angereichert wird. Über drei kreisförmige Öffnungen 4 kann die mit Duftstoff versetzte Luft in den Fahrgastraum eines Fahrzeugs eintreten. Die Öffnungen 4 und die Öffnungsschlitze 13 sind dabei sowohl in der Abdeckung 32 als auch in jeweiligen Wandteilen, welche vom Unterteil 31 oder dem Träger 35 nach oben abragen ausgebildet, so dass ein Luftdurchgang nur dann erfolgt, wenn die Abdeckung so verdreht ist, dass die jeweiligen Öffnungen zur Deckung kommen. Wird die Abdeckung 32 weiter verdreht, können die Öffnungen 4 verschlossen werden.

Die Spannungsversorgung des Lüfters wird wiederum durch eine oder mehrere Batterien, welche in einer Batterieaufnahme im Abdeckteil 32 oder im Unterteil 31 angeordnet sind gewährleistet (nicht dargestellt). Optional kann über die Verdrehung der Abdeckung 32 ein Schleifkontakt unterbrochen werden, so dass die Spannungsversorgung des Lüfters bei verschlossenen Öffnungen 4 unterbrochen ist.

Die gesamte Beduftungsvorrichtung 1 ist so gestaltet, dass sie in einen Cupholder eines Fahrzeugs einsetzbar ist., wobei der Boden des Unterteils 31 des Gehäuses 2 bevorzugt auf die gleiche Art mit Dämpfungselementen 5 wie im ersten Ausführungsbeispiel ausgeführt ist. Des weiteren ist in der Abdeckung 32 ein Betriebsanzeigegerät und im Gehäuse 2 eine Steuereinheit wie bereits zum ersten Ausführungsbeispiel beschrieben, vorgesehen. Die Intensität der Duftstoffabgabe lässt sich sowohl durch die entsprechende Gebläsestärke als auch manuell durch entsprechende Verdrehung der Abdeckung 32 einstellen. Es sei ausdrücklich erwähnt, dass auch das zweite Ausführungsbeispiel mit zwei oder mehreren in einem Winkel zueinander angeordneten Duftkartuschen einsetzbar ist, so dass auch verschiedene Duftnoten einstellbar sind.

## Patentansprüche

1. Beduftungsvorrichtung (1) für ein Kraftfahrzeug, mit zumindest einer Duftstoffaufnahmevorrichtung (6), einem Lüfterrad (19), einem Antriebsmotor für das Lüfterrad und einer elektrischen Energieversorgungseinrichtung (7) für den Antriebsmotor des Lüfterrads (19), die in einem Gehäuse (2) angeordnet sind, wobei die Energieversorgungseinrichtung (7) den Antriebsmotor des Lüfterrades (19) unabhängig vom Energieversorgungssystem des Kraftfahrzeugs mit Energie versorgt, wobei die Duftstoffaufnahmevorrichtung (6) eine im Gehäuse (2) der Beduftungsvorrichtung feststehende Einfassung (12) und einen im Gehäuse (2) drehbar angeordneten Aufnehmer (11) aufweist, wobei der Aufnehmer (11) ein Aufnahmefach oder mehrere Aufnahmefächer für eine oder mehrere Duftstoffkartuschen (14) aufweist, **dadurch gekennzeichnet, dass** in einem Bereich zwischen dem Aufnehmer (11) und der Einfassung (12) ein oder mehrere Dichtelemente angeordnet sind.

2. Beduftungsvorrichtung nach Anspruch 1, **dadurch gekennzeichnet, dass** Energieversorgungseinrichtung (7) zumindest eine oder mehrere, vorzugsweise wiederaufladbare Batterien umfasst.

3. Beduftungsvorrichtung nach Anspruch 1 oder 2, **dadurch gekennzeichnet, dass** im zumindest einen Gehäuse (2) eine Steuereinheit (8) für den Antriebsmotor des Lüfterrades (19) angeordnet ist.

4. Beduftungsvorrichtung nach Anspruch 3, **dadurch gekennzeichnet, dass** die Steuereinheit (8) mit einem Zeitschalter versehen ist.

5. Beduftungsvorrichtung nach Anspruch 3 oder 4, **dadurch gekennzeichnet, dass** die Steuereinheit (8) mit einem Bewegungsmelder versehen ist.

6. Beduftungsvorrichtung nach einem der vorhergehenden Ansprüche, **dadurch gekennzeichnet, dass** an dem zumindest einen Gehäuse (2) ein Anschluss für ein Ladekabel vorgesehen ist.

7. Beduftungsvorrichtung nach einem der vorhergehenden Ansprüche, **dadurch gekennzeichnet, dass** das zumindest eine Gehäuse (2) im Wesentlichen zylindrisch ausgeführt ist.

8. Beduftungsvorrichtung nach einem der vorhergehenden Ansprüche, **dadurch gekennzeichnet, dass** das zumindest eine Gehäuse (2) eine Abdeckung (3, 32) aufweist.

9. Beduftungsvorrichtung nach Anspruch 8, **dadurch gekennzeichnet, dass** die Abdeckung (3, 32) an einem Gehäuseunterteil (31) des Gehäuses (2), insbesondere durch eine Schraub- und/oder Schnappverbindung montier- und demontierbar ist.

10. Beduftungsvorrichtung nach einem der vorhergehenden Ansprüche, **dadurch gekennzeichnet, dass** das Gehäuse (2) ein Unterteil (31) aufweist, das mit der Abdeckung (32) über einen Verbindungsring (34) verbunden ist

11. Beduftungsvorrichtung nach Anspruch 10, **dadurch gekennzeichnet, dass** der Verbindungsring (34) ein Gewindering ist.

12. Beduftungsvorrichtung nach einem der vorhergehenden Ansprüche, **dadurch gekennzeichnet, dass** die Intensität des Duftaustrags durch Verdrehung der Abdeckung (32) einstellbar ist.

## Claims

1. Fragrancing device (1) for a motor vehicle, having at least one fragrance acceptance device (6), one fan wheel (19), one drive motor for the fan wheel and one electric energy supply equipment (7) for the drive motor of the fan wheel (19), arranged in a housing (2), wherein the energy supply equipment (7) supplies the drive motor of the fan wheel (19) with energy independent of the energy supply system of the motor vehicle, wherein the fragrance acceptance device (6) has an edge (12) fixed within the housing (2) of the fragrancing device and an acceptor (11) rotatably arranged within the housing (2), wherein the acceptor (11) has one storage compartment or several storage compartments for one or more fragrance cartridges (14), **characterised in that** one or more sealing elements are arranged in an area between the acceptor (11) and the edge (12).

2. Fragrancing device according to claim 1, **characterised in that** the energy supply equipment (7) comprises at least one or more preferably rechargeable batteries.

3. Fragrancing device according to claim 1 or 2, **characterised in that** a control unit (8) for the drive motor of the fan wheel (19) is arranged in the at least one housing (2).

4. Fragrancing device according to claim 3, **characterised in that** the control unit (8) is provided with a time switch.

5. Fragrancing device according to claim 3 or 4, **characterised in that** the control unit (8) is provided with a motion detector.

6. Fragrancing device according to one of the preceding claims, **characterised in that** a connection for a charging cable is provided on the at least one housing (2).

7. Fragrancing device according to one of the preceding claims, **characterised in that** the at least one housing (2) is substantially cylindrical.

8. Fragrancing device according to one of the preceding claims, **characterised in that** the at least one housing (2) has a cover (3, 32).

9. Fragrancing device according to claim 8, **characterised in that** the cover (3, 32) is mountable and demountable on a housing base (31) of the housing (2), especially through a screw and/or snap connection.

10. Fragrancing device according to one of the preceding claims, **characterised in that** the housing (2) has a base (31) which is connected to the cover (32) via a connecting ring (34).

11. Fragrancing device according to claim 10, **characterised in that** the connecting ring (34) is a threaded ring.

12. Fragrancing device according to one of the preceding claims, **characterised in that** the intensity of the fragrance discharge is adjustable by turning the cover (32).

## Revendications

1. Dispositif servant à parfumer (1), prévu pour un véhicule automobile et comprenant au moins un dispositif récepteur de parfum (6), une roue de ventilateur (19), un moteur d'entraînement pour la roue de ventilateur et un dispositif d'alimentation en énergie électrique (7) pour le moteur d'entraînement de la roue de ventilateur (19), tous ces composants étant disposés dans un boîtier (2), où le dispositif d'alimentation en énergie (7) alimente en énergie le moteur d'entraînement de la roue de ventilateur (19) indépendamment du système d'alimentation en énergie du véhicule automobile, où le dispositif récepteur de parfum (6) présente une bordure enveloppante (12) fixe dans le boîtier (2) du dispositif servant à parfumer et un élément récepteur (11) disposé en pouvant tourner dans le boîtier (2), où l'élément récepteur (11) présente un compartiment de réception ou plusieurs compartiments de réception pour une ou plusieurs cartouches de parfum (14), **caractérisé en ce qu'**un ou plusieurs éléments d'étanchéité est ou sont disposé(s) dans une zone comprise entre l'élément récepteur (11) et la bordure enveloppante (12).

2. Dispositif servant à parfumer selon la revendication 1, **caractérisé en ce que** le dispositif d'alimentation en énergie (7) comprend au moins une ou plusieurs batteries, de préférence rechargeable(s).

3. Dispositif servant à parfumer selon la revendication 1 ou 2, **caractérisé en ce qu'**une unité de commande (8) prévue pour le moteur d'entraînement de la roue de ventilateur (19) est disposée dans le boîtier (2) au moins au nombre de un.

4. Dispositif servant à parfumer selon la revendication 3, **caractérisé en ce que** l'unité de commande (8) est dotée d'un interrupteur à minuterie.

5. Dispositif servant à parfumer selon la revendication 3 ou 4, **caractérisé en ce que** l'unité de commande (8) est dotée d'un indicateur de mouvement.

6. Dispositif servant à parfumer selon l'une quelconque des revendications précédentes, **caractérisé en ce qu'**un raccordement pour un câble de recharge est prévu sur le boîtier (2) au moins au nombre de un.

7. Dispositif servant à parfumer selon l'une quelconque des revendications précédentes, **caractérisé en ce que** le boîtier (2) au moins au nombre de un est réalisé de façon pratiquement cylindrique.

8. Dispositif servant à parfumer selon l'une quelconque des revendications précédentes, **caractérisé en ce que** le boîtier (2) au moins au nombre de un présente un couvercle (3, 32).

9. Dispositif servant à parfumer selon la revendication 8, **caractérisé en ce que** le couvercle (3, 32) est montable et démontable sur une partie inférieure (31) du boîtier (2), en particulier grâce à un assemblage vissé et / ou par encliquetage.

10. Dispositif servant à parfumer selon l'une quelconque des revendications précédentes, **caractérisé en ce que** le boîtier (2) présente une partie inférieure (31) qui est reliée au couvercle (32) par une bague d'assemblage (34).

11. Dispositif servant à parfumer selon la revendication 10, **caractérisé en ce que** la bague d'assemblage (34) est une bague filetée.

12. Dispositif servant à parfumer selon l'une quelconque des revendications précédentes, **caractérisé en ce que** l'intensité de la diffusion de parfum est réglable en tournant le couvercle (32).
